Europäisches Patentamt

European Patent Office (11) Numéro de publication: **0 322 293**

Office européen des brevets **A1**

(12) ## DEMANDE DE BREVET EUROPEEN

(21) Numéro de dépôt: **88403226.9**

(51) Int. Cl.⁴: **A 61 B 5/14**

(22) Date de dépôt: **16.12.88**

(30) Priorité: **21.12.87 FR 8717811**

(43) Date de publication de la demande:
**28.06.89 Bulletin 89/26**

(84) Etats contractants désignés:
**AT BE CH DE ES FR GB GR IT LI LU NL SE**

(71) Demandeur: **BIOVALLEES Société Anonyme:**
**F-43150 Monastier sur Gazelle (FR)**

(72) Inventeur: **Moisson, Claude**
**2, Place du Tertre**
**F-28000 Nogent-le-Phaye (FR)**

(74) Mandataire: **Derambure, Christian et al**
**BUGNION ASSOCIES 55, rue Boissonade**
**F-75014 Paris (FR)**

(54) Dispositif de prélèvement et d'analyse du sang.

(57) L'invention concerne un dispositif de prélèvement et d'analyse du sang. Il comporte un autopiqueur (1), un réservoir (2) de bandelettes, ces bandelettes comportant une zone réactive, un réflectomètre (6). De plus le réservoir (2) est solidaire de l'autopiqueur (1) et le dispositif comporte des moyens (7,8) permettant au repos de solidariser le réflectomètre (6) et l'autopiqueur (1).

FIG. 1

## Description

## DISPOSITIF DE PRELEVEMENT ET D'ANALYSE DU SANG

Les analyses du sang sont pratiquées de plus en plus fréquemment aussi bien en milieu hospitalier, que par les médecins par, les infirmières ou les laboratoires spécialisés ou encore le patient lui-même.

Il est possible de déterminer des concentrations sanguines en composants divers tels que le sucre, le cholestérol et en général toutes les ormones en utilisant des bandelettes réactives dont la couleur est analysée après la réaction.

Ces bandelettes comportent une petite surface recouverte d'un composé comprenant un réactif spécifique du corps dont on cherche à évaluer la concentration et des réactifs secondaires qui sont des pigments colorés selon la concentration du corps à évaluer.

L'emploi de ces bandelettes réactives est bien connu et se décompose de la façon suivante :

On dépose tout d'abord une goutte fraîchement prélevée au bout du doigt ou à l'oreille sur la surface réactive. Ensuite, on laisse la réaction se dérouler pendant un temps déterminé variable. Puis on évacue le sang soit par lavage soit par essuyage et on attend pendant une nouvelle période de temps également déterminée. Enfin, on effectue une mesure de la couleur à l'aide d'un réflectomètre. Le résultat obtenu est comparé directement à une abaque et converti en concentration de produit.

La mise en oeuvre de ce procédé nécessite plusieurs instruments.

Le prélèvement de la goutte de sang est effectué à l'aide d'un autopiqueur. Ce dispositif est destiné à donner à une fléchette munie d'une pointe stérile un mouvement rapide qui lui permet de percer superficiellement la peau du doigt ou de l'oreille sur lequel il a été placé. Cette piqûre permet la collecte de la goutte de sang nécessaire à l'analyse. La fléchette est généralement protégée au repos par le corps de l'autopiqueur. Son mouvement est obtenu par l'action d'un ressort principal préalablement bandé. Un ressort secondaire comprimé lors de l'action du ressort principal assure le retour de la lancette à son emplacement initial à l'intérieur du corps de l'autopiqueur. Plusieurs modèles d'autopiqueurs sont actuellement disponibles, le corps du plus récent a la forme d'un stylo.

Les bandelettes sont généralement distribuées dans un tube.

Le contrôle des temps de réaction avant et après l'essuyage du sang nécessite l'utilisation d'un chronomètre.

La mesure de la couleur de la surface réactive est réalisée par un réflectomètre. Il comporte une source lumineuse monochrome et une diode réceptrice ainsi que des moyens permettant de transformer l'intensité de la lumière réfléchie en la valeur du paramètre à mesurer. Il comporte un dispositif d'affichage permettant de visualiser le résultat.

Certains réflectomètres incorporent un chronomètre ce qui permet de diminuer le nombre d'instruments nécessaires à la réalisation des mesures.

L'objectif de l'invention est la conception d'un dispositif de prélèvement et d'analyse du sang qui permet de réaliser l'ensemble des opérations mentionnées plus haut d'une manière très simple et qui est facilement portable.

C'est également un objectif de l'invention de proposer un appareil qui puisse être manipulé d'une seule main par exemple pendant que l'autre est mobilisée par la ponction.

A cet effet, le dispositif de prélèvement et d'analyse du sang de l'invention comporte un autopiqueur, un réservoir de bandelettes, ces bandelettes comportant une zone réactive, et un réflectomètre. Le réservoir est solidaire de l'autopiqueur et le dispositif comporte des moyens permettant au repos de solidariser le réflectomètre et l'autopiqueur.

Selon un mode de réalisation préféré de l'invention, l'autopiqueur et le réflectomètre comportent des éléments complémentaires permettant leur solidarisation par emboîtement pendant la lecture par le réflectomètre de la couleur de la zone réactive.

L'invention sera décrite en détail en référence aux figures dans lesquelles :

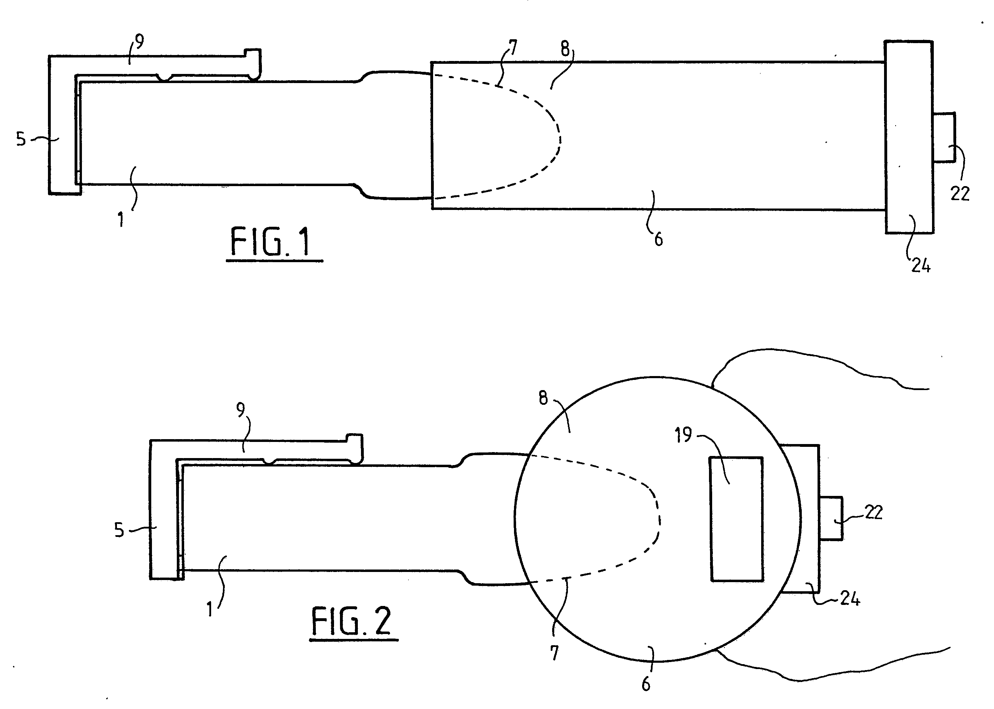

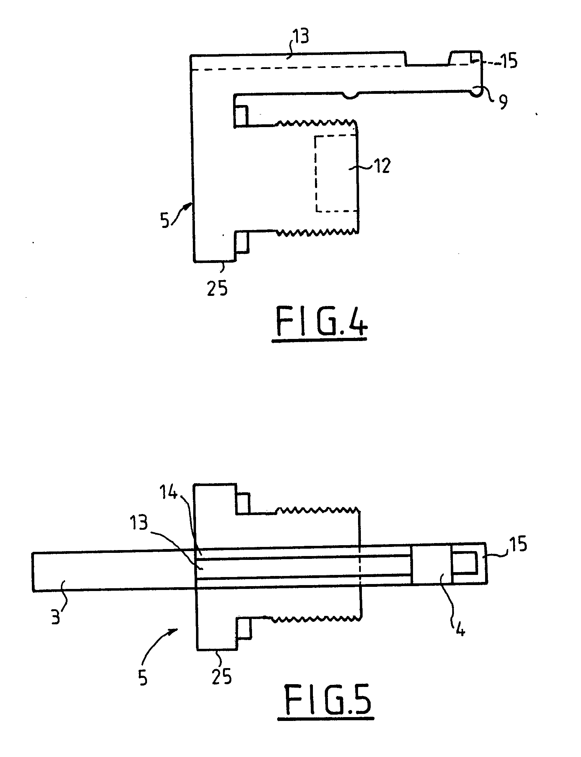

. La figure 1 représente une vue en coupe du dispositif solidarisé en position de repos selon une première forme de réalisation

. la figure 2 représente une vue en coupe du dispositif solidarisé en position de repos selon une deuxième forme de réalisation,

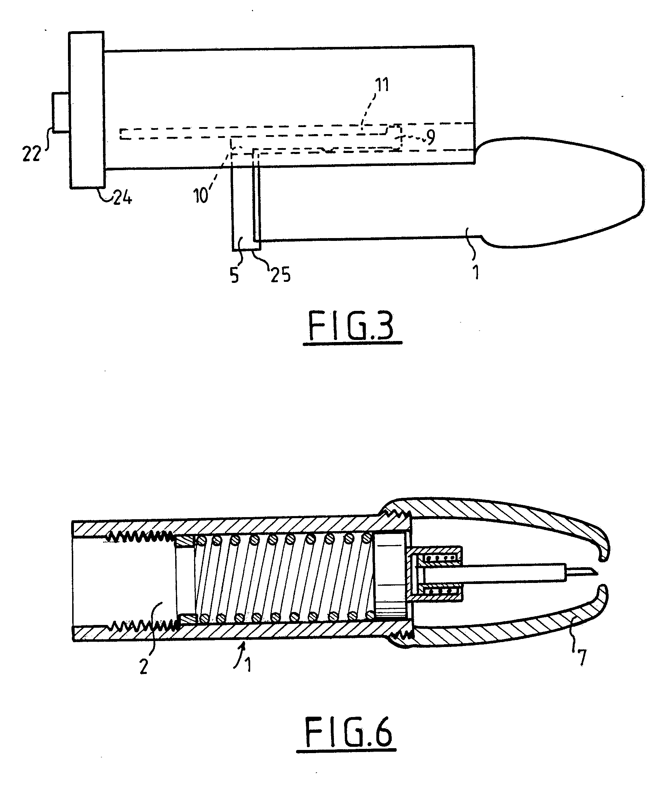

. la figure 3 représente une vue en coupe du dispositif solidarisé en position de lecture,

. la figure 4 représente une vue en coupe verticale du bouchon jetable,

. la figure 5 représente une vue du dessus du bouchon jetable, et d'une bandelette réactive,

. la figure 6 représente une vue en coupe de l'élément autopiqueur,

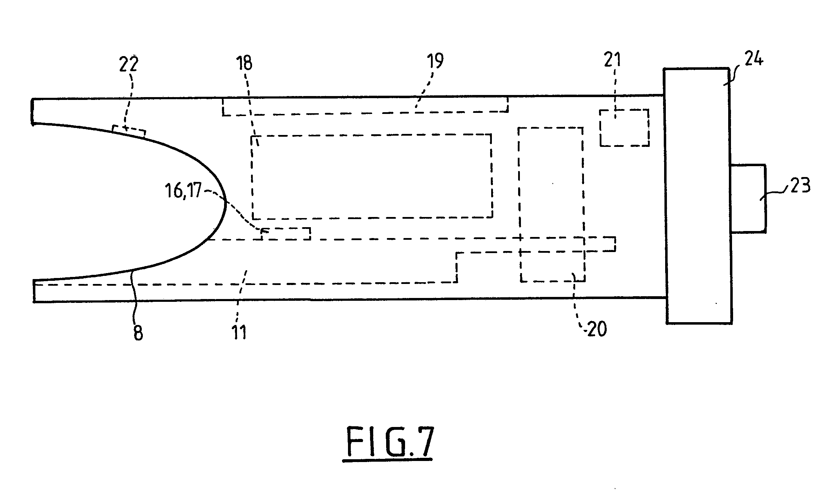

. la figure 7 représente une vue du dessus du réflectomètre.

Le dispositif de prélèvement et d'analyse du sang comporte un autopiqueur 1, un réservoir 2 de bandelettes. Ces bandelettes comportent un support mince 3 généralement en matière plastique souple sur laquelle est déposé un réactif constituant une zone réactive 4. Le réservoir 2 est solidaire de l'autopiqueur 1. Il est constitué par un logement prévu à l'intérieur même du corps de l'autopiqueur 1. Ce réservoir 2 est fermé par un bouchon 5. Le réflectomètre 6 et l'autopiqueur 1 ont des formes complémentaires constituant des moyens permettant au repos de solidariser l'un avec l'autre. Ces éléments 7 et 8 sont de préférence légèrement coniques et l'un au moins d'entre eux étant susceptible de faible déformation élastique permettant la solidarisation de l'un avec l'autre par coincement. Les extrémités 7 et 8 de l'autopiqueur peuvent également comporter d'autres moyens de solidarisation tels que des pas de vis complémentaires ou une gorge pouvant coopérer par clipsage avec une protubérance.

Ainsi, le dispositif de l'invention constitue au repos une entité unique dont le maniement est très simple. Le réflectomètre 6 comme l'autopiqueur 1 peuvent être de forme allongée sensiblement cylindrique de telle sorte que le dispositif de l'invention est alors facilement logeable dans une poche à la manière d'un stylo. Le bouchon 5 peut d'ailleurs être munie d'une barette 8 facilitant l'accrochage du dispositif à l'intérieur d'une poche.

Dans un autre mode de réalisation, représenté sur la figure 2 le réflectomètre peut avoir une forme plus compacte sensiblement circulaire au carré et être muni d'un collier qui permet à l'utilisateur de l'accrocher autour de son cou.

L'autopiqueur 1 et le réflectomètre 6 comportent également des éléments complémentaires 10 et 11 permettant leur solidarisation par emboîtement pendant la lecture par le réflectomètre 6 de la couleur de la zone réactive 4 de la bandelette 2. Le dispositif est ainsi facilement manipulable aussi bien lorsqu' il est en repos que pendant la phase de lecture.

Les réactifs portés par la bandelette sont fortement sensibles à l'humidité. Afin d'améliorer leur conservation, le réservoir 2 comporte un bouchon jetable 5 auquel est incorporé un dissicateur 12. Ce bouchon 5 peut être fourni en même temps que les recharges de bandelettes de telle sorte que le dissicateur 12 est toujours actif.

Le bouchon jetable 5 est muni d'une rainure 13 destinée à recevoir et à maintenir la bandelette 3. Cette rainure 13 comporte des rebords 14 sous lesquels peut s'engager la bandelette qui est ainsi parfaitement maintenue. La zone réactive 4 est précisément positionnée lorsque la bandelette 3 vient en butée au fond 15 de la rainure 13.

Lors du prélèvement, il est ainsi possible de placer une bandelette 3 dans la rainure 13 l'autopiqueur 1 est alors maintenu d'une main afin de faire le prélèvement sur l'autre main. Après la piqûre, une goutte de sang est déposée sur la bandelette 3 à l'endroit de la zone réactive 4.

De préférence, la rainure 13 est dégagée au niveau de la zone réactive 4. C'est-à-dire que les rebords 14 ainsi que les flancs de la rainures 13 ne sont interrompus à ce niveau. Le dépôt de la goutte de sang sur la zone réactive 4 est ainsi facilitée de même que son essuiement après le temps de réaction nécessaire.

Selon cette disposition, le support de la bandelette 3 est constitué par le bouchon jetable 5. Cela est particulièrement avantageux car à la suite d'un certain nombre de prélèvements il est inévitable que le support de la bandelette soit souillé. De cette manière, il est régulièrement renouvelé.

Selon un mode de réalisation préféré, la barette 9 du bouchon jetable 5 porte la rainure 13.

Selon un mode de réalisation particulièrement avantageux, l'élément 10 de l'autopiqueur complémentaire de l'élément 11 du réflectomètre et permettant la solidarisation de ces deux éléments par emboîtement pendant la lecture est constitué par la barette 9. L'élément 11 du réflectomètre est alors constitué par une gorge. L'emboîtement de la barette 9 dans la gorge 11 permet ainsi un positionnement précis de la zone réactive 4 de la bandelette 3 par rapport à la source lumineuse 16 et à la diode réceptrice 17. La mesure est donc ainsi réalisée dans les conditions optimales.

Le réflectomètre 6 comporte de plus un système de traitement 18, un système d'affichage 19, un chronomètre 20, un dispositif sonore 21.

Le dispositif de traitement comporte un convertisseur traduisant l'intensité de la lumière réfléchie pour une longueur d'ondes en un temps, un calcul logique qui permet de transformer ce temps en la valeur du paramètre à mesurer par comparaison avec les données inscrites dans une mémoire morte et correspondant à une abaque.

Cette mémoire morte peut en outre réaliser d'autres fonctions telles que la mémorisation des derniers résultats, de l'heure à laquelle ils ont été obtenus et éventuellement la communication avec un calculateur plus puissant chargé de traiter ces résultats.

L'emboîtement de l'autopiqueur et du réflectomètre en position de repos assure la coupure du circuit électrique du réflectomètre par un interrupteur 22. Inversement, leur dissociation assure sa mise sous tension. Le bouton 23 est utilisé pour déclencher le chronomètre et l'alarme sonore est mise en route lorsque le temps écoulé correspond aux durées préprogrammées nécessaires à la réaction avant et après l'essuyage. La molette 24 permet le réglage du chronomètre.

Selon un mode de réalisation préféré, le bouchon jetable 5 comporte une face plane 25 qui assure la stabilité du dispositif tant en position de repos qu'en position de lecture lorsqu'il est posé sur une surface plane horizontale.

## Revendications

1) Dispositif de prélèvement et d'analyse du sang du type comportant un autopiqueur (1), un réservoir (2) de bandelettes, ces bandelettes comportant une zone réactive, un réflectomètre (6), caractérisé en ce que le réservoir (2) est solidaire de l'autopiqueur (1) et qu'il comporte des moyens (7, 8) permettant au repos de solidariser le réflectomètre (6) et l'autopiqueur (1).

2) Dispositif selon la revendication 1, caractérisé en ce que l'autopiqueur (1) et le réflectomètre (6) comportent des éléments complémentaires (10, 11) permettant leur solidarisation par emboîtement pendant la lecture par le réflectomètre (6) de la couleur de la zone réactive (4).

3) Dispositif selon la revendication 2, caractérisé en ce que l'autopiqueur (1) et le réflectomètre (6) comportent des moyens assurant la mise sous tension du réflectomètre (6) lors de leur solidarisation pendant la lecture et seulement dans cette position.

4) Dispositif selon l'une quelconque des revendications 1 à 3, caractérisé en ce que le réservoir (2) comporte un bouchon jetable (5) auquel est incorporé un dessicateur.

5) Dispositif selon l'une quelconque des revendications 1 à 4, caractérisé en ce que le réservoir (2) comporte un bouchon jetable (5) muni d'une rainure destinée à recevoir la bandelette avant que celle-ci ait reçu le sang prélevé.

6) Dispositif selon la revendication 5, caractérisé en ce que la rainure (13) du bouchon (5) assure le positionnement précis de la zone réactive (4) de la bandelette et est interrompu à son niveau de manière à faciliter son essuiement.

7) Dispositif selon l'une quelconque des revendications 5 et 6, caractérisé en ce que la rainure (13) est portée par une barette (9) faisant partie du bouchon (5).

8) Dispositif selon la revendication 7, caractérisé en ce que le réflectomètre (6) comporte une gorge destinée à recevoir la barette (9) et permettant de le positionner par rapport à la zone réactive (4) de la bandelette lors de son utilisation.

9) Dispositif selon l'une quelconque des revendications 4 à 8, caractérisé en ce que le bouchon (5) comporte une face plane sur laquelle peut reposer l'ensemble du dispositif au repos et/ou lors de l'utilisation du réflectomètre (6).

10) Dispositif selon l'une quelconque des revendications 1 à 9, caractérisé en ce que le réflectomètre (6) comporte un système de lecture (16, 17), un système de traitement (18), un système d'affichage (19), un chronomètre (20), un dispositif sonore et que le système de traitement (18) permet l'affichage direct de la concentration recherchée.

11) Dispositif selon la revendication 10, caractérisé en ce que le système de traitement comporte un microprocesseur et une mémoire morte comportant les données permettant de convertir l'information fournie par le système de lecture en la concentration recherchée.

FIG.1

FIG.2

FIG.4

FIG.5

FIG.3

FIG.6

FIG.7

EP 0 322 293 A1

Office européen
des brevets

**RAPPORT DE RECHERCHE EUROPEENNE**

Numero de la demande

EP 88 40 3226

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 199 484 (AUDIO BIONICS INC.) <br> * Résumé; colonne 8, ligne 1 - colonne 12, ligne 11; figure 1-5 * <br> --- | 1,2,10, 11 | A 61 B 5/14 |
| A | FR-A-2 508 305 (G.S. SLAMA) <br> * Page 3, ligne 15 - page 6, ligne 17; figure 1-5 * <br> ----- | 1,2,4 | |

**DOMAINES TECHNIQUES RECHERCHES (Int. Cl.4)**

A 61 B 5/00
A 61 B 17/00
G 01 N 33/00

Le présent rapport a été établi pour toutes les revendications

| Lieu de la recherche | Date d'achèvement de la recherche | Examinateur |
|---|---|---|
| BERLIN | 17-02-1989 | WEIHS J.A. |